# EUROPÄISCHE PATENTSCHRIFT

(11) **EP 2 987 529 B1**
(45) Veröffentlichungstag und Bekanntmachung des Hinweises auf die Patenterteilung: **14.12.2016**
(21) Anmeldenummer: 15167604.6
(22) Anmeldetag: 13.05.2015
(51) Int. Cl.: A61N 1/372, A61N 1/375, A61N 1/05, A61B 17/34, A61B 17/00

(54) **IMPLANTAT MIT EINER FIXIEREINRICHTUNG UND EINFÜHRVORRICHTUNG MIT EINEM IMPLANTAT**
IMPLANT COMPRISING A FIXING DEVICE, AND INSERTION APPARATUS COMPRISING AN IMPLANT
IMPLANT DOTÉ D'UN DISPOSITIF DE FIXATION ET DISPOSITIF D'INTRODUCTION D'UN IMPLANT

(30) Priorität: 19.08.2014 US 201462038840 P
(43) Veröffentlichungstag der Anmeldung: 24.02.2016
(73) Patentinhaber: BIOTRONIK SE & Co. KG, 12359 Berlin (DE)
(72) Erfinder: Dörr, Thomas, 12437 Berlin (DE); Diebold, Michael, 12169 Berlin (DE)
(74) Vertreter: Galander, Marcus

(56) Entgegenhaltungen:
- WO-A2-2013/098644
- DE-A1- 2 840 307
- US-A1- 2005 004 644
- US-A1- 2011 106 145
- US-A1- 2011 238 077
- US-A1- 2012 330 392

## Beschreibung

Die Erfindung betrifft ein Implantat zum Einführen in den menschlichen oder tierischen Körper, mit einer Fixiereinrichtung und eine Einführvorrichtung mit einem Implantat nach den Oberbegriffen der unabhängigen Ansprüche.

Es ist bekannt, Implantate zum Einführen in den menschlichen oder tierischen Körper, wie beispielsweise Endokard- und Epikardelektroden, mit einer so genannten Fixierhelix zu fixieren. Das Einschrauben der Fixierhelix erfolgt dabei entweder durch eine Drehung des gesamten Implantats oder ein Herausschrauben der Fixierhelix durch einen Kardanantrieb innerhalb der Elektrodenleitung.

Dieses Vorgehen eignet sich jedoch nur bedingt für eine Implantation in schmalen und schlecht zugänglichen Regionen, wie z.B. auf dem Myokard, sofern kein direkter Zugang zum Implantationsort möglich ist.

WO 2013/098644 zeigt ein medizinisches Implantat mit wandelförmiger Fixierungseinrichtung und einer Bestätigungseinrichtung, die mit der Fixierungseinrichtung verbunden ist und durch ihre Drehbewegung dem Fixierungselement eine Längsbewegung verleiht.

Der Erfindung liegt die Aufgabe zugrunde, ein sicher fixierbares Implantat für schmale oder schlecht zugängliche Implantationsorte zu schaffen.

Eine weitere Aufgabe besteht darin, eine Einführvorrichtung für ein solches Implantat zu schaffen.

Die Aufgaben werden erfindungsgemäß durch die Merkmale der unabhängigen Ansprüche gelöst. Günstige Ausgestaltungen und Vorteile der Erfindung ergeben sich aus den weiteren Ansprüchen, der Beschreibung und der Zeichnung.

Es wird ein medizinisches Implantat mit einer Fixiereinrichtung mit wenigstens einem Fixierelement zum Fixieren des Implantats an einem Implantationsort vorgeschlagen, wobei das Implantat einen Riemenantrieb aufweist, der eine Betätigungseinrichtung in eine Drehbewegung versetzt, wobei die Betätigungseinrichtung mit der Fixiereinrichtung gekoppelt ist und die Drehbewegung in eine Längsbewegung der Fixiereinrichtung umsetzt.

Vorteilhaft kann ein Fixierelement des Implantats, insbesondere eine Fixierhelix, auch in sehr schmalen Gewebezwischenräumen implantiert werden und sicher in das zu implantierende Gewebe eingedreht werden. Ferner kann die genaue Umdrehungsanzahl für das Herausdrehen einer Fixierhelix sichergestellt werden.

Als Riemen des Riemenantriebs kann zweckmäßigerweise ein reißfester, hinreichend dünner Faden vorgesehen sein, der auf einfache Weise in einer Einführvorrichtung geführt werden kann.

Vorteilhaft kann ein Herausdrehen einer Fixierhelix aus einem flach unter das Perikard verschiebbaren oder vergleichbaren Implantats erfolgen. Dieser Antrieb erfolgt mittels eines um die Fixierhelix oder das Implantat gewickelten Fadens, so dass dieser bei Zug die Fixierhelix oder das Implantat drehen und sich gegebenenfalls dann vom Implantat lösen kann, wenn die Fixierhelix komplett ausgedreht ist. Optional kann auch ein simultanes Herausdrehen von zwei Fixierhelices in entgegengesetzter Drehrichtung erfolgen, so dass das Implantat sich nicht selbst losdrehen kann, sondern am Implantationsort sicher verankert ist.

Besonders vorteilhaft kann das medizinische Implantat in Form eines epikardialen oder endokardialen Pulsgenerators oder eines Neurostimulators eingesetzt sein.

Nach einer vorteilhaften Ausgestaltung kann ein Riemen des Riemenantriebs die Betätigungseinrichtung umschlingen, insbesondere mehrfach mit einer definierten Zahl von Windungen umschlingen, wobei die Betätigungseinrichtung eine rotationssymmetrische Kontaktfläche für den Riemen des Riemenantriebs aufweist. Durch Festlegen des Riemens an der Kontaktfläche kann die Betätigungseinrichtung rotiert werden.

Nach einer vorteilhaften Ausgestaltung kann ein Riemen des Riemenantriebs lösbar an der Betätigungseinrichtung so angeordnet sein, dass sich der Riemen von der Betätigungseinrichtung antriebsmäßig entkoppelt oder löst, wenn eine vorgegebene Zahl von Umdrehungen der Betätigungseinrichtung erreicht ist. Dadurch kann eine definierte Zahl an Umdrehungen der Betätigungseinrichtung erreicht werden, die wiederum eine definierte Vorschubbewegung des Fixierelements und damit einen definierten Überstand des Fixierelements über das Implantatgehäuse hinaus vorgeben. Damit kann eine definierte Tiefe erreicht werden, in die sich das Fixierelement in das Gewebe am Implantationsort eingräbt.

Nach einer vorteilhaften Ausgestaltung kann der Riemenantrieb ausgebildet sein, die Betätigungseinrichtung in beide Drehrichtungen zu bewegen. Vorteilhaft können zwei Riementeile vorgesehen sein, die an der Betätigungseinrichtung so befestigt sind, dass sich zwei freie Enden des Riemens von der Betätigungseinrichtung weg erstrecken. Wird an einem Ende gezogen, dreht sich die Betätigungseinrichtung in eine Richtung, wird am anderen Ende gezogen, dreht sich die Betätigungseinrichtung in die dazu entgegengesetzte Richtung.

Nach einer vorteilhaften Ausgestaltung kann die Fixiereinrichtung mehrere Fixierelemente aufweisen. Vorteilhaft können die mehreren Fixierelemente simultan bewegbar sein, wenn die Betätigungseinrichtung dreht. Dadurch kann eine besonders sichere Verankerung des Implantats am Implantationsort bewirkt werden.

Nach einer vorteilhaften Ausgestaltung können die mehreren Fixierelemente zumindest in ausgefahrenem Zustand gegeneinander verschiebbar und/oder verkippbar sein, so dass diese zueinander flexibel angeordnet sind. Sind die mehreren Fixierelemente im bestimmungsgemäßen Gebrauch zueinander flexibel angeordnet, kann sichergestellt werden, dass diese eine durch ein sich bewegendes Gewebe ergebende Relativbewegung, z.B. bei einem Myokard, ausgleichen können.

Nach einer vorteilhaften Ausgestaltung kann die Fixiereinrichtung mit einer Verriegelung gekoppelt sein, die zumindest eine Längsbewegung bei gelöstem oder entkoppeltem Riemenantrieb unterbindet. Die Verriegelung kann eine weitere Drehung der Fixierhelix immer dann verhindern, wenn der Riemen vollständig herausgezogen ist, etwa in der Art einer Sperrklinke, die durch den Riemen zunächst blockiert ist.

Nach einer vorteilhaften Ausgestaltung kann der Riemen oder können die Riemen des Riemenantriebs formschlüssig mit der Betätigungseinrichtung gekoppelt sein. Vorteilhaft kann der Riemen oder können die Riemen und die Betätigungseinrichtung ineinandergreifende Strukturen aufweisen, die bei der Drehbewegung der Betätigungseinrichtung miteinander zusammenwirken. Der Riemen kann in bestimmten Bereichen mechanische Konturelemente aufweisen, die so nur in einem definierten Bereich eine Kopplung mit der Betätigungseinrichtung herstellen, beispielsweise in Form kleiner Kugeln oder Knoten, die in Vertiefungen der Kontaktfläche zwischen Betätigungseinrichtung und Riemen eingreifen können. Damit lässt sich der Kontakt zwischen Betätigungseinrichtung und Riemen verbessern und eine gezielte Krafteinleitung zum Drehen der Betätigungseinrichtung bewirken.

Nach einer vorteilhaften Ausgestaltung kann der Riemen aus resorbierbarem Material gebildet sein. Der Riemen kann somit problemlos am Implantat verbleiben.

Nach einer vorteilhaften Ausgestaltung kann die Fixiereinrichtung eine oder mehrere Fixierhelices aufweisen. Diese lassen sich besonders einfach über eine Drehbewegung in eine Vorschubrichtung bewegen.

Nach einem weiteren Aspekt der Erfindung wird eine Einführeinrichtung für ein medizinisches Implantat vorgeschlagen, wobei das medizinische Implantat eine Fixiereinrichtung mit wenigstens einem Fixierelement zum Fixieren des Implantats an einem Implantationsort umfasst, wobei das Implantat einen Riemenantrieb aufweist, der eine Betätigungseinrichtung in eine Drehbewegung versetzt, wobei die Betätigungseinrichtung mit der Fixiereinrichtung gekoppelt ist und die Drehbewegung in eine Längsbewegung der Fixiereinrichtung umsetzt.

Vorteilhaft kann in der Einführeinrichtung eine Aufnahme für das Implantat vorgesehen sein und der Riemenantrieb über die Einführvorrichtung betätigbar sein. Der Riemenantrieb ist Bestandteil des Implantationswerkzeuges, das derart geformt ist, dass das Implantat mit der Fixiereinrichtung zunächst vollständig aufgenommen wird und bei Betätigung des Mechanismus diese im gleichen Maße aus dem Implantationswerkzeug ausführt und in das Gewebe eintreibt. Im Falle einer Fixierhelix wird diese im gleichen Maße aus dem Implantationswerkzeug herausgeschraubt und in das Gewebe eingeschraubt.

Die Erfindung ist nachfolgend beispielhaft, anhand von in Zeichnungen dargestellten Ausführungsbeispielen, näher erläutert. Es zeigen in schematischer Darstellung:
- Fig. 1a, 1b: als Schnittansicht ein Implantat mit einer Fixierhelix vor dem Fixieren (Figur 1a) und nach dem Fixieren (Figur 1b);
- Fig. 2a, 2b: eine Draufsicht auf eine Unterseite (Figur 2a) und eine Seitenansicht (Figur 2b) eines Implantats nach einer Ausgestaltung der Erfindung;
- Fig. 3: eine Seitenansicht eines Implantats mit Fixierhelix nach einer weiteren Ausgestaltung der Erfindung; und
- Fig. 4: eine Seitenansicht einer Einführvorrichtung mit einem Implantat mit Fixierhelix mit teilweise aufgeschnittener Halterung nach einem unabhängigen Aspekt der Erfindung; und
- Fig. 5: eine Ausgestaltung eines Riemenantriebs nach einer Ausführungsform der Erfindung;
- Fig. 6: eine Seitenansicht eines Implantats nach einer Ausgestaltung der Erfindung.

In den Figuren sind funktionell gleiche oder gleich wirkende Elemente jeweils mit denselben Bezugszeichen beziffert. Die Figuren sind schematische Darstellungen der Erfindung. Sie bilden nicht spezifische Parameter der Erfindung ab. Weiterhin geben die Figuren lediglich typische Ausgestaltungen der Erfindung wieder und sollen die Erfindung nicht auf die dargestellten Ausgestaltungen beschränken.

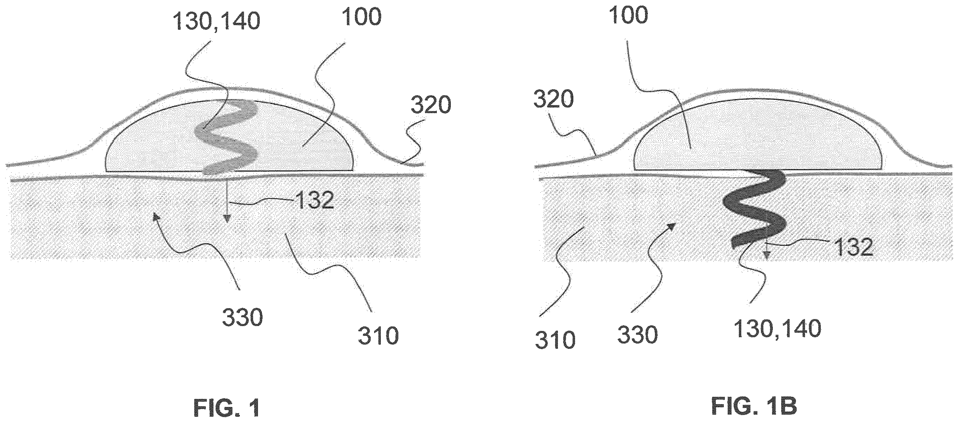

Die Figuren 1a und 1b zeigen zur Erläuterung der Erfindung als Schnittansicht ein Implantat 100 mit einem als Fixierhelix ausgebildeten Fixierelement 140 einer Fixiereinrichtung 130 vor dem Fixieren (Figur 1a) und nach dem Fixieren (Figur 1b) in einem Implantationsort 330. In der Figur ist das Implantat 100 beispielhaft als epikardialer Pulsgenerator ausgebildet, welcher zunächst mittels einer nicht dargestellten Einführvorrichtung zwischen Myokard 310 und Perikard 320 positioniert wird (Figur 1a) und anschließend im Gewebe des Myokard 310 des Implantationsorts 330 mittels eines als Fixierhelix ausgebildeten Fixierelements 140 fixiert wird, welches aus diesem Implantat 100 in einer Längsrichtung 132 ausgeschraubt wird (Figur 1a).

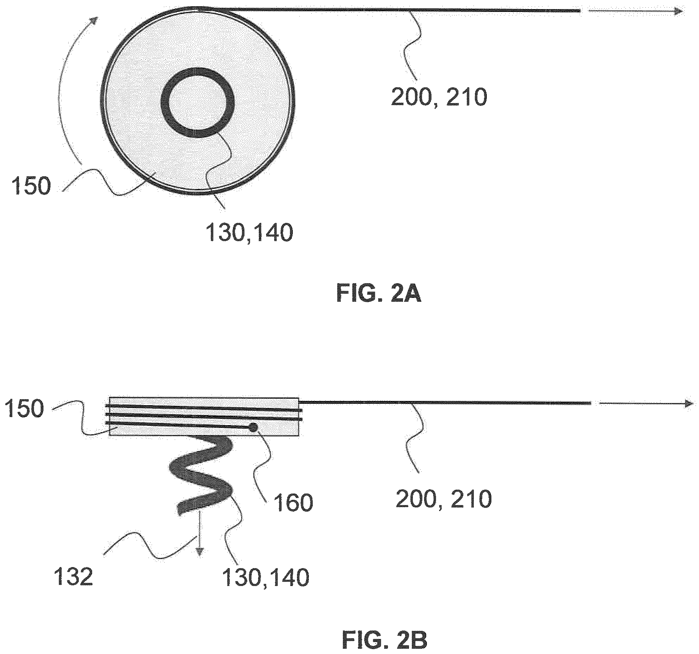

Wie in den Figuren 2a in Draufsicht und 2b in Seitenansicht näher erläutert ist, weist das Implantat 100 hierzu einen Riemenantrieb 200 auf, der eine Betätigungseinrichtung 150 in eine Drehbewegung versetzt, wobei die Betätigungseinrichtung 150 mit der Fixiereinrichtung 130 gekoppelt ist und die Drehbewegung in eine Längsbewegung der Fixiereinrichtung 130 umsetzt. Bei einer Fixierhelix als Fixierelement 140, welche in üblicher Weise ein Gewinde mit einer gegebenen Steigung aufweist, ist die Längsbewegung mit einer Drehung der Fixierhelix entsprechend der Steigung des Gewindes der Fixierhelix gekoppelt. Die Betätigungseinrichtung ist dabei im Implantat entweder an einer Achse längsverschiebbar angebracht, so dass die Fixierhelix beim Einschrauben in das Körpergewebe die Betätigungseinrichtung auf der Achse verschiebt. Alternativ ist die Achse mit einem Gewinde entsprechender Steigung ausgeführt, die diese Längsverschiebung der Betätigungseinrichtung aktiv vornimmt.

Der Riemenantrieb 200 umschlingt eine rotationssymmetrische Kontaktfläche der Betätigungseinrichtung 150 mit einem fadenartigen Riemen 210. Die Zahl der Windungen gibt zweckmäßigerweise die maximal mögliche Auslenkung des Fixierelements 140 in Längsrichtung 132 (in der Figur mit einem Pfeil gekennzeichnet) vor. Der Riemen 210 ist an der Kontaktfläche an einem Punkt 160 fixiert. Die Betätigungseinrichtung 150 ist drehbar gelagert (nicht dargestellt). Wird am freien Ende des Riemens 210 gezogen, dreht sich die Betätigungseinrichtung 150, und das Fixierelement 140 kann mit einem Gegengewinde oder dergleichen zusammenwirken und in Längsrichtung 132 bewegt werden.

Vorteilhaft ist der Riemen 210 des Riemenantriebs 200 lösbar an der Betätigungseinrichtung 150 so angeordnet, dass sich der Riemen 210 von der Betätigungseinrichtung 150 löst, wenn eine vorgegebene Zahl von Umdrehungen der Betätigungseinrichtung 150 erreicht ist oder sich dieser ablöst, wenn das Fixierelement 140 vollständig ausgeschraubt ist. Der Riemen 210 kann vorteilhaft aus resorbierbarem Material gebildet sein und im Gewebe verbleiben.

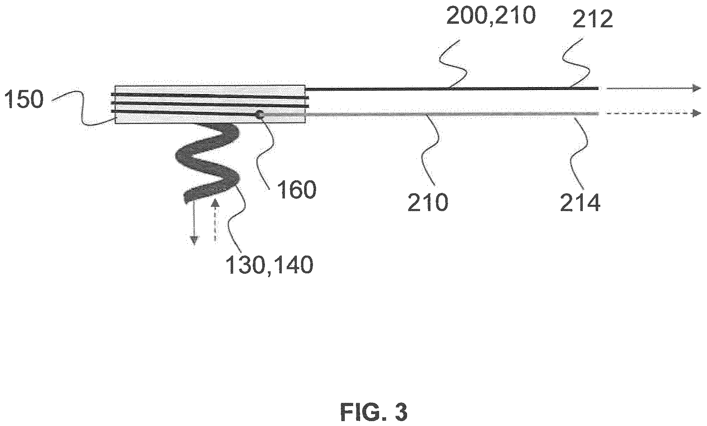

In der Figur 3 ist eine Variante zu der Anordnung in den Figuren 2a, 2b gezeigt. Hier ist der Riemen 210 so angebracht, dass zwei Riemenenden 212, 214 sich von der Betätigungseinrichtung 150 weg erstrecken. Je nachdem, welches der Enden 212, 214 mit Zug beaufschlagt wird, dreht die Betätigungseinrichtung 150 in die eine oder andere Drehrichtung. Dadurch kann die Betätigungseinrichtung 150 in beide Drehrichtungen bewegt werden, so dass ein Einschrauben der Fixierhelix in das Gewebe mit Zug an dem Riemenende 212 und mit Zug an dem anderen Riemenende 214 ein Zurückschrauben möglich ist.

Vorzugsweise ist der Riemen 210 an einem Punkt 160 derart fixiert, dass dieser sich von der Betätigungseinrichtung 150 ablösen kann, wenn das Fixierelement 140 vollständig herausgedreht ist und das erste Teilstück des Riemens 210 herausgezogen wird.

Dabei kann der Riemen 210 derart befestigt sein, dass dieser sich von der Betätigungseinrichtung 150 abkoppelt, wenn die erforderliche Anzahl von Umdrehungen zum Einschrauben in das Gewebe erfolgt ist, jedoch auch wieder einkoppelt, wenn der Mechanismus zum Zurückschrauben betätigt wird und gleichzeitig so ausgeführt wird, dass nach dem Einschrauben ein Ablösen des gesamten fadenförmigen Riemens 210 gegeben ist, so dass der gesamte Riemen 210 vollständig herausgezogen wird.

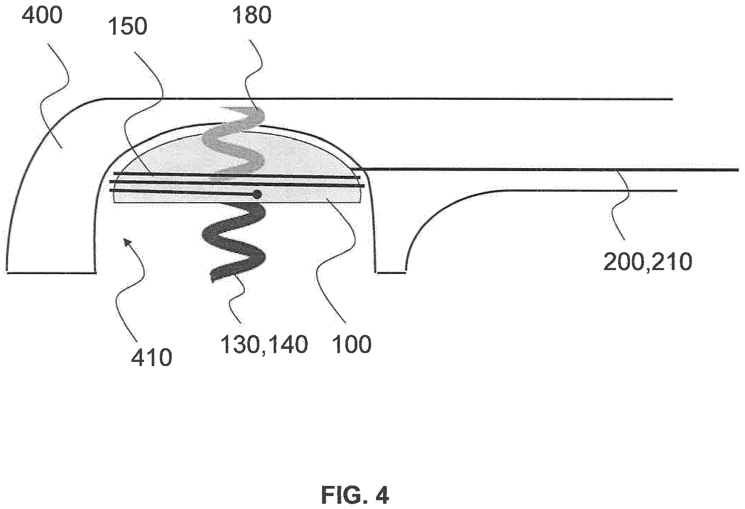

Figur 4 zeigt in einer Seitenansicht einer Einführvorrichtung 400 mit einem medizinischen Implantat 100 und einer Fixiereinrichtung 130, mit teilweise aufgeschnittener Halterung nach einem unabhängigen Aspekt der Erfindung. In der Einführeinrichtung 400 ist eine Aufnahme 410 für das Implantat 100 vorgesehen. Die Fixiereinrichtung 130 weist eine Fixierhelix als Fixierelement 140 auf.

Ein Riemenantrieb 200 ist über die Einführvorrichtung 400 betätigbar, wobei ein fadenförmiger Riemen 210 durch die Halterung zum proximalen Ende der Einführvorrichtung 300 führt. Dadurch, dass das Implantat 100 in der Aufnahme 410 vollständig aufgenommen ist, kann das Implantat 100 in einen Gewebezwischenraum eingebracht werden, ohne dass das feste, hier als Fixierhelix ausgebildete Fixierelement 140 den Zugang verhindert.

Bei Zug an dem Riemen 210 zur Betätigungseinrichtung 150 wird das gesamte rotationssymmetrische Implantat 100 im Implantationswerkzeug 400 gedreht. Dieses beinhaltet zusätzlich eine helixförmige Führung 180 des Implantats 100 mit annähernd der gleichen Steigung wie die des Fixierelements 140 am Implantat 100, um so das Implantat 100 zeitgleich aus der Einführvorrichtung 400 herauszuschrauben und in das Gewebe einzuschrauben.

In Abhängigkeit vom Zielgewebe kann durch die unterschiedliche Steigung beider Helices 180, 140 die Anschraubkraft in das Gewebe kontrolliert eingestellt werden.

In Analogie zum Ausführungsbeispiel in Figur 3 kann auch hier ein Mechanismus zum Zurückschrauben vorgesehen sein.

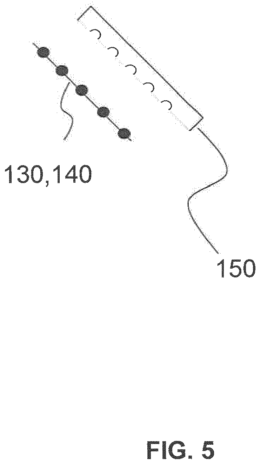

Figur 5 zeigt eine Ausgestaltung eines Riemenantriebs 200 nach einer Ausführungsform der Erfindung, bei welcher der Riemen 210 des Riemenantriebs 200 formschlüssig mit der Betätigungseinrichtung 150 gekoppelt ist. Der Riemen 210 und die Betätigungseinrichtung 150 weisen ineinandergreifende Strukturen auf, die bei der Drehbewegung der Betätigungseinrichtung 150 miteinander zusammenwirken.

Die Erfindung ermöglicht es, ein Implantat 100 mittels eines Fixierelements 140, insbesondere einer Fixierhelix, auch an schwierig zugänglichen Positionen sicher und einfach zu fixieren.

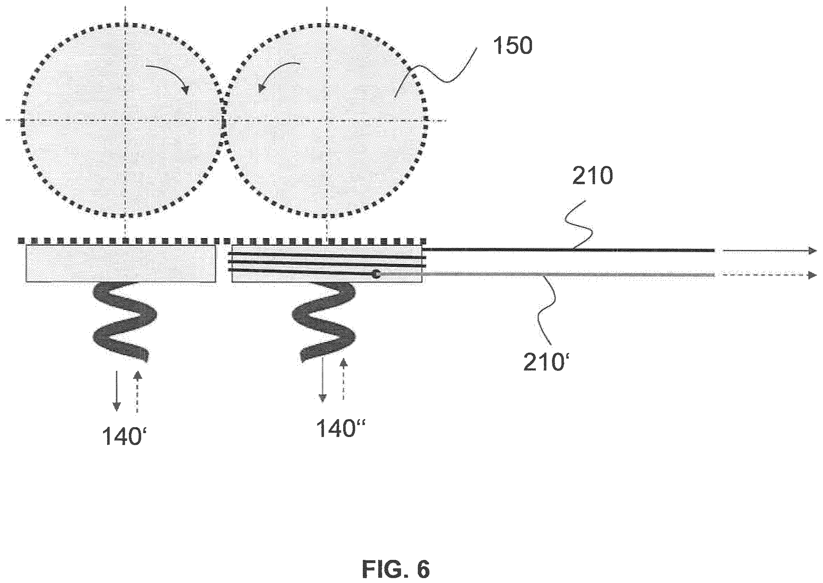

Figur 6 zeigt eine Ausgestaltung eines Implantats mit zwei Fixierelementen 140' und 140" die von einem Riemen 210, 210' über die Betätigungseinrichtung 150 bewegbar sind. Beispielhaft dient der Riemen 210 um die Helices im Gewebe zu verankern und der Riemen 210' um die Helices aus dem Gewebe herauszuschrauben. Bevorzugt wird dabei das die Fixierelemente 140' und 140" von gegenläufige Helices gebildet werden und die Betätigungsvorrichtung 150 derart ausgestaltet ist, dass die Helices sich in entgegengesetzter Richtung in das Gewebe einschrauben, also eine im Uhrzeigersinn, die andere gegen den Uhrzeigersinn. Beim einem Herausschrauben der Helices müssen diese auch entgegengesetzt zueinander herausgedreht werden, also eine gegen den Uhrzeigersinn, die andere im Uhrzeigersinn. Damit wird erreicht, dass sich das Implantat nicht durch Bewegungen, insbesondere Herzbewegungen, aus dem Gewebe schrauben kann.

## Patentansprüche

1. Medizinisches Implantat (100) mit einer Fixiereinrichtung (130) mit wenigstens einem Fixierelement (140) zum Fixieren des Implantats (100) an einem Implantationsort (330), wobei das Implantat (100) einen Riemenantrieb (200) aufweist, der eine Betätigungseinrichtung (150) in eine Drehbewegung versetzt, wobei die Betätigungseinrichtung (150) mit der Fixiereinrichtung (130) gekoppelt ist und die Drehbewegung in eine Längsbewegung der Fixiereinrichtung (130) umsetzt.

2. Medizinisches Implantat nach Anspruch 1, wobei ein Riemen (210,) des Riemenantriebs (200) die Betätigungseinrichtung (150) umschlingt, und wobei die Betätigungseinrichtung (150) eine rotationssymmetrische Kontaktfläche für den Riemen des Riemenantriebs (200) aufweist.

3. Medizinisches Implantat nach Anspruch 1 oder 2, wobei ein Riemen (210) des Riemenantriebs (200) lösbar an der Betätigungseinrichtung (150) so angeordnet ist, dass sich der Riemen (210) von der Betätigungseinrichtung (150) antriebsmäßig entkoppelt oder löst, wenn eine vorgegebene Zahl von Umdrehungen der Betätigungseinrichtung (150) erreicht ist.

4. Medizinisches Implantat nach einem der vorhergehenden Ansprüche, wobei der Riemenantrieb (200) ausgebildet ist, die Betätigungseinrichtung (150) in beide Drehrichtungen zu bewegen.

5. Medizinisches Implantat nach einem der vorhergehenden Ansprüche, wobei die Fixiereinrichtung (130) mehrere Fixierelemente (140) aufweist.

6. Medizinisches Implantat nach Anspruch 5, wobei die mehreren Fixierelemente (140) simultan bewegbar sind, wenn die Betätigungseinrichtung (150) dreht.

7. Medizinisches Implantat nach Anspruch 5 oder 6, wobei die Fixierelemente (140) zumindest in ausgefahrenem Zustand gegeneinander verschiebbar und/oder verkippbar sind.

8. Medizinisches Implantat nach einem der vorhergehenden Ansprüche, wobei die Fixiereinrichtung (130) mit einer Verriegelung gekoppelt ist, die zumindest eine Längsbewegung bei gelöstem Riemenantrieb (200) unterbindet.

9. Medizinisches Implantat nach einem der vorhergehenden Ansprüche, wobei der Riemen (210) des Riemenantriebs (200) formschlüssig mit der Betätigungseinrichtung (150) gekoppelt ist.

10. Medizinisches Implantat nach einem der vorhergehenden Ansprüche, wobei der Riemen (210) und die Betätigungseinrichtung (150) ineinandergreifende Strukturen aufweisen, die bei der Drehbewegung der Betätigungseinrichtung (150) miteinander zusammenwirken.

11. Medizinisches Implantat nach einem der vorhergehenden Ansprüche, wobei der Riemen (210) aus resorbierbarem Material gebildet ist.

12. Medizinisches Implantat nach einem der vorhergehenden Ansprüche, wobei die Fixiereinrichtung (130) eine oder mehrere Fixierhelices aufweist.

13. Medizinisches Implantat nach einem der vorhergehenden Ansprüche in Form eines epikardialen oder endokardialen Pulsgenerators oder eines Neurostimulators.

14. Einführeinrichtung (400) für ein medizinisches Implantat (100) nach einem der vorhergehenden Ansprüche, wobei eine Aufnahme (410) für das Implantat (100) vorgesehen ist und der Riemenantrieb (200) über die Einführvorrichtung (400) betätigbar ist.

## Claims

1. A medical implant (100) comprising a fixing device (130) having at least one fixing element (140) for fixing the implant (100) at a site of implantation (330), wherein the implant (100) has a belt drive (200), which rotates an actuation device (150), wherein the actuation device (150) is coupled to the fixing device (130) and converts the rotary movement into a longitudinal movement of the fixing device (130).

2. The medical implant as claimed in Claim 1, wherein a belt (210) of the belt drive (200) loops around the actuation device (150), and wherein the actuation device (150) has a rotationally symmetrically contact face for the belt of the belt drive (200).

3. The medical implant as claimed in Claim 1 or 2, wherein a belt (210) of the belt drive (200) is arranged detachably on the actuation device (150), such that the belt (210) is decoupled or detached in terms of drive from the actuation device (150) when a predefined number of revolutions of the actuation device (150) is reached.

4. The medical implant as claimed in any one of the preceding claims, wherein the belt drive (200) is configured to move the actuation device (150) in both directions of rotation.

5. The medical implant as claimed in any one of the preceding claims, wherein the fixing device (130) comprises a plurality of fixing elements (140).

6. The medical implant as claimed in Claim 5, wherein the plurality of fixing elements (140) are movable simultaneously when the actuation device (150) rotates.

7. The medical implant as claimed in Claim 5 or 6, wherein the fixing elements (140) are displaceable and/or tiltable with respect to one another at least in the extended state.

8. The medical implant as claimed in any one of the preceding claims, wherein the fixing device (130) is coupled to a locking mechanism, which at least prevents a longitudinal movement when the belt drive (200) is detached.

9. The medical implant as claimed in any one of the preceding claims, wherein the belt (210) of the belt drive (200) is coupled in a form-fitting manner to the actuation device (150).

10. The medical implant as claimed in any one of the preceding claims, wherein the belt (210) and the actuation device (150) have structures engaging with one another, which cooperate with one another when the actuation device (150) rotates.

11. The medical implant as claimed in any one of the preceding claims, wherein the belt (210) is formed from resorbable material.

12. The medical implant as claimed in any one of the preceding claims, wherein the fixing device (130) comprises one or more fixing helices.

13. The medical implant as claimed in any one of the preceding claims in the form of an epicardial or endocardial pulse generator or a neurostimulator.

14. An insertion device (400) for a medical implant (100) as claimed in any one of the preceding claims, wherein a receptacle (410) for the implant (100) is provided and the belt drive (200) is actuatable via the insertion apparatus (400).

## Revendications

1. Implant médical (100) doté d'un dispositif de fixation (130) avec au moins un élément de fixation (140) pour la fixation de l'implant (100) à un lieu d'implantation (330), où l'implant (100) présente un entraînement à courroie (200) qui transpose le dispositif d'actionnement (150) dans un mouvement de rotation, où le dispositif d'actionnement (150) est couplé avec le dispositif de fixation (130) et le mouvement de rotation se transforme en un déplacement longitudinal du dispositif de fixation (130).

2. Implant médical selon la revendication 1, où une courroie (210) entoure l'entraînement à courroie (200) du dispositif d'actionnement (150), et où le dispositif d'actionnement (150) présente une surface de contact de symétrie par rotation pour la courroie de l'entraînement à courroie (200).

3. Implant médical selon la revendication 1, ou 2, où une courroie (210) de l'entraînement à courroie (200) est disposée de manière amovible sur le dispositif d'actionnement (150) de sorte que la courroie (210) est découplée ou détachée du point de vue de l'entraînement du dispositif d'actionnement (150), lorsqu'un nombre prédéfini de tours du dispositif d'actionnement (150) est atteint.

4. Implant médical selon l'une des revendications précédentes, où l'entraînement à courroie (200) est conçu pour déplacer le dispositif d'actionnement (150) dans deux directions de rotation.

5. Implant médical selon l'une des revendications précédentes, où le dispositif de fixation (130) présente plusieurs éléments de fixation (140).

6. Implant médical selon la revendication 5, où les nombreux éléments de fixation (140) peuvent être déplacés simultanément lorsque le dispositif d'actionnement (150) est en rotation.

7. Implant médical selon la revendication 5, ou 6, où les éléments de fixation (140) peuvent être déplacés et/ou basculés les uns par rapport aux autres au moins à l'état non fonctionnel.

8. Implant médical selon l'une des revendications précédentes, où le dispositif de fixation (130) est couplé avec un verrouillage qui empêche au moins un déplacement longitudinal pour l'entraînement à courroie (200) non fonctionnel.

9. Implant médical selon l'une des revendications précédentes, où la courroie (210) de l'entraînement à courroie (200) est couplée par complémentarité des formes avec le dispositif d'actionnement (150).

10. Implant médical selon l'une des revendications précédentes, où la courroie (210) et le dispositif d'actionnement (150) présentent des structures s'imbriquant qui agissent conjointement lors du mouvement en rotation du dispositif d'actionnement (150).

11. Implant médical selon l'une des revendications précédentes, où la courroie (210) est constituée d'un matériau résorbable.

12. Implant médical selon l'une des revendications précédentes, où le dispositif de fixation (130) ^présente une ou plusieurs hélices de fixation.

13. Implant médical selon l'une des revendications précédentes sous la forme d'un générateur de pulsation épicardiaques ou endocardiaques ou d'un neurostimulateur.

14. Dispositif d'insertion (400) pour un implant médical (100) selon l'une des revendications précédentes, où un logement (410) est prévu pour l'implant (100) et l'entraînement à courroie (200) peut être actionné par l'intermédiaire du dispositif d'insertion (400).
